# EUROPEAN PATENT APPLICATION

(11) **EP 2 133 114 A1**
(43) Date of publication of application: **16.12.2009**
(21) Application number: 08739672.7
(22) Date of filing: 02.04.2008
(51) Int. Cl.: A61M 25/00, A61F 2/82, A61F 2/84

(54) **STENT DELIVERY SYSTEM**

(30) Priority: 05.04.2007 JP 2007099675
(71) Applicant: Kaneka Corporation, Kita-ku Osaka-shi, Osaka 530-8288 (JP)
(72) Inventor: SHIMADA, Tamotsu, Settsu-shi Osaka 566-0072 (JP)
(74) Representative: Gille Hrabal Struck Neidlein Prop Roos
(86) International application number: PCT/JP2008/056561
(87) International publication number: WO 2008/126737

(57) **Abstract**

A stent delivery system comprises a delivery catheter with a balloon and a stent. The stent delivery system is **characterized in that** when a force to move the stent in the axial direction of the catheter is applied to the stent, a holding mechanism is formed in a part of the balloon to prevent the stent from falling down. According to this invention, the stent delivery system that is capable of preventing the stent from falling down and has excellent track ability can be provided without requiring complicated manufacturing processes.

## Description

### Technical Field

The present invention relates to a stent delivery system including a delivery catheter having a balloon and a stent.

### Background Art

Stents have been used widely for dilation of stricture sites formed in vascular systems in human body, and assuring the preservation of the routes. A stent is placed in a catheter called delivery catheter as it is folded and inserted into and expanded in the body.

There are two kinds of stents according to the expanding mode. One is a balloon-expandable stent that is expanded by expansion force applied by a balloon expanded with a pressurized fluid by a balloon catheter. The other is a self-expandable stent that expands by itself, for example as it is made of a shape-memory alloy.

In the case of the balloon-expandable stent, the stent is folded and tightened around a shaft before installation into a delivery catheter. However, the steut-retention force applied from outside is not sufficient for fixation of the stent therein, and thus there was a concern about the stent being caught in a bent region of vascular system such as blood vessel, and thus the stent being dislocated or removed from its original site on balloon catheter by the frictional resistance during insertion, for example by friction between the hemostasis valve and the guide catheter or friction in meandering vascular system. The stent, if dislocated from its original site, cannot be expanded uniformly and thus, cannot dilate the stricture site effectively The stent, if it falls off, is dangerous because it possibly remains in the body. Methods of preventing such dislocation and fall-off of a stent are disclosed in the following prior-art literatures.

Patent Document 1 discloses that it is possible to prevent fall-off of the stent by forming multiple projections on the balloon surface and thus making the stent captured by the projections or embedded thereon.
However, it is difficult by the method to form the projections on the external and internal surfaces of the balloon, and there is a concern that capturing and embedding of the complicatedly shaped stent by the projections may be unstable.

In Patent Document 2, dislocation of the stent is suppressed, as the stent is captured by the ridges formed on the balloon surface in the circumferential direction. Also in the method, the crimping profile (external diameter of stent after installation as folded) becomes enlarged because the balloon is made pleat-shaped as the ridges are formed on balloon in the circumferential direction. The increase in crimping profile leads to expansion of the diameter of the shaft in the catheter distal end region and deterioration in flexibility of the region.

In Patent Document 3, the catheter has a retaining sleeve and fixes the stent while covering the terminal thereof. There is a concern in the method that the sleeve formed on the catheter may lead to increase in profile and also in production man-power.

In Patent Document 4, the stent is held as it is covered with a folded balloon in the stent terminal region. The method leads to increase in profile because the balloon is placed on the stent. It is also difficult to place the stent in the folded balloon.

In these prior arts, the stent is fixed by capturing by projections on the balloon surface or by a retention mechanism such as sleeve. If the stent is placed as folded by such a method, the profile becomes enlarged compared to the case where there is no mechanism for prevention of stent dislocation. The increase in profile leads to deterioration in flexibility, causing problems such as deterioration in trackability performance due to increase in passage resistance in blood vessel and increase in complexity of the steps for installing the mechanism, and increase in man power for the production steps.
Patent Document 1: WO 00/57815
Patent Document 2: WO 2002/066096
Patent Document 3: WO 90/05554
Patent Document 4: WO 00/078249

### Disclosure of the Invention

### Technical Problems to be Solved

It is an object of the invention to provide a stent delivery system that forms a stent-retention mechanism and thus prevent fall-off of the stent when a force for moving the stent in the catheter axial direction is applied. Means to Solve the Problems

The present invention, which achieved the object above, has the following characteristics.

[1] A stent delivery system including a delivery catheter having a balloon and a stent, **characterized in that** the balloon has a straight-tube region and a tapered region formed in at least one of the distal and proximal end-sided regions thereof, and at least a part of the balloon forms a stent-retention mechanism.

[2] The stent delivery system of [1], wherein the stent-retention mechanism is not formed when the stent is placed in the delivery catheter as the diameter thereof is reduced, and is formed when a force for moving the stent in the catheter axial direction is applied.

[3] The stent delivery system of [2], wherein the stent-retention mechanism returns back to its original state when the force for moving the stent in the catheter axial direction is removed.

[4] The stent delivery system of [2] or [3], wherein the stent-retention mechanism is formed on at least one terminal area of the distal- and proximal-end sides of the stent placed as crimped.

[5] The stent delivery system of [4], wherein the stent-retention mechanism is formed on at least an area of the straight-tube region of the balloon where the crimped stent is not placed and the tapered region.

[6] The stent delivery system of [4], wherein the stent-retention mechanism serves as a stopper preventing fall-off of the stent by forming at least one pleat.

[7] The stent delivery system of [4], wherein the tapered region is longer than the region of the balloon straight-tube region where the stent is not placed, on at least one terminal area of the stent distal- and proximal-end sides.

[8] The stent delivery system of [4], wherein the stent-retention mechanism is formed on the distal-end side of the crimped stent, when a force for moving the stent in the distal-end side is applied.

[9] The stent delivery system of [4], wherein the straight-tube region of the balloon has regions where the stent is not placed both on the distal- and proximal-end sides and the distal end-sided region is longer than the proximal end-sided region.

Other characteristics and advantages of the present invention will become more obvious with the following embodiments and drawings.

### Effects of the Invention

The present invention provides a stent delivery system that is superior in trackability performance and that can be produced easily without any complicated steps because a retention mechanism for prevention of fall-off of the stent is formed when a force moving the stent in the catheter axial direction is applied and the crimped stent is simply placed on the folded balloon when the force is not applied.

### Brief Description of Drawings

Figure 1 is a schematic view illustrating a high-speed exchange balloon catheter among common balloon catheters.
Figure 2 is a schematic view illustrating an over-the-wire balloon catheter among common balloon catheters.
Figure 3 is a schematic view illustrating a balloon in a stent delivery catheter in an embodiment of the present invention.
Figure 4 is a schematic view illustrating a balloon in a folded state in a stent delivery catheter in an embodiment of the present invention.
Figure 5 is a schematic view illustrating a distal end area in the stent delivery catheter in an embodiment of the present invention.
Figure 6 shows an example of a stent delivery catheter containing a stent-retention mechanism formed according to an embodiment.
Figure 7 shows another example of a stent delivery catheter containing a stent-retention mechanism formed according to an embodiment.
Figure 8 shows yet another example of a stent delivery catheter containing a stent-retention mechanism formed according to an embodiment.
Figure 9 shows yet another example of a stent delivery catheter containing a stent-retention mechanism formed according to an embodiment.
Figure 10 shows yet another example of a stent delivery catheter containing a stent-retention mechanism formed according to an embodiment.
Figure 11 shows an example of a position of a stent delivery catheter having a stent-retention mechanism crimped according to an embodiment.
Figure 12 shows an example of the other shape of the stent delivery catheter according to an embodiment.
Figure 13 shows another example of the other shape of the stent delivery catheter according to an embodiment.
Figure 14 shows yet another example of the other shape of the stent delivery catheter according to an embodiment.
Figure 15 shows yet another example of the other shape of the stent delivery catheter according to an embodiment.
Figure 16 is a sectional view of the stent delivery catheter along a line A-B in Figure 15.

### Brief Description of Numerals

1: Balloon
2: Balloon straight-tube region
2a: Distal end-sided region of straight-tube region where the stent is not placed
2b: Straight-tube region where the stent is placed
2c: Proximal end-sided region of straight-tube region where the stent is not placed
3: Balloon tapered region
3a: Distal end-sided tapered region
3b: Proximal end-sided tapered region
4: Stent
5: Stent-retention mechanism
6: Friction layer
7: Inner tube
8: Outer tube
9: Hub
11: Distal-end side
12: Proximal-end side
13: External force
14: Vertex of the balloon blade

### Best Mode for Carrying Out the Invention

Hereinafter, preferred embodiments of the catheter and the balloon according to the present invention will be described with reference to drawings.

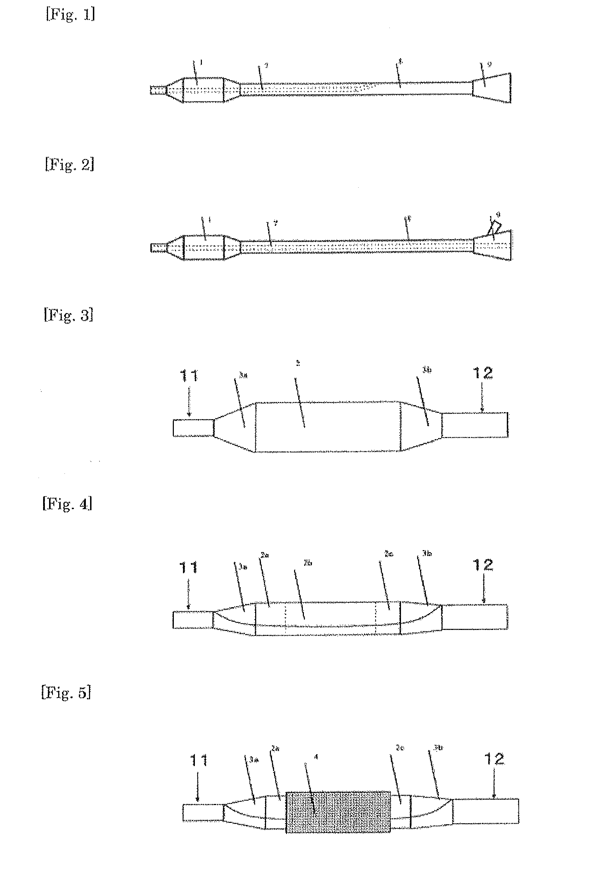

The catheter in an embodiment of the present invention may have any of the structures of a known high-speed exchange catheter shown in Figure 1 or an over-the-wire catheter shown in Figure 2. The catheter in the embodiment is not particularly limited if it has a balloon on a distal-end side of the catheter, a tube or lumen for supply of pressurized fluid into the balloon, and a tube or lumen for insertion of a guide wire, and it is a stent delivery catheter in which at least a part of the medical balloon forms a stent-retention mechanism.

Hereinafter, an embodiment of the present invention (balloon installed in the catheter, and the like) will be described in detail, by using a coaxial high-speed exchange stent delivery catheter having a structure of coaxially-arranged outer and inner tubes as an example.

### (1) Configuration of entire balloon

As shown in Figure 3, a medical balloon 1 in an embodiment has a cylindrical straight-tube region 2, a conical distal-end tapered region 3a in the distal-end side of the straight tube region, and a comical proximal-end tapered region 3b in the proximal-end side of the straight tube region. As shown in Figure 4, when the stent is placed as the balloon is folded, the straight-tube region 2 preferably has a shape having a straight-tube region 2b on which a crimped stent is placed, a distal-end-sided straight-tube region 2a where the stent is not placed, and a proximal-end-sided straight-tube region 2c where the stent is not placed. A cylindrical shape and a conical shape were exemplified respectively for the straight-tube region and tapered regions, but the shape are not limited to these shapes, and any shape known to those who are skilled in the art may be used.

### (2) Stent-retention mechanism

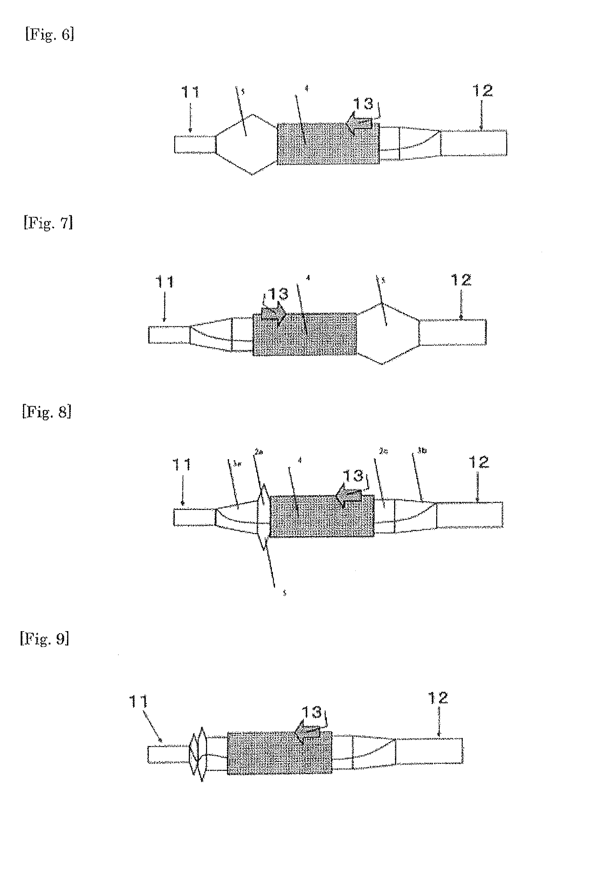

In the present embodiment, as shown in Figure 5, the crimped stent 4 is placed on the folded balloon. As shown in Figures 6 and 7, when an external force 13 is applied to the crimped stent for movement in the catheter axial direction, a stent-retention mechanism 5 is formed. The stent-retention mechanism, as stopper, can prevent fall-off of the stent. When the external force 13 is removed, the retention mechanism returns back to its original shape, but the return may not be complete.

In a more preferred embodiment, as shown in Figure 8, the stent-retention mechanism 5 may be formed by at least a part of the balloon.

The stent-retention mechanism is preferably formed only when an external force 13 is applied to the stent for movement in the catheter axial direction. Preferably, in this case, a profile of a catheter diameter remains constant under normal condition and the trackability performance is the same as that in the case where the stent is simply tightened around the catheter. Such a configuration has an advantage that the profile may not be increased, in contrast to the case where an additional mechanism is formed in the balloon and stent regions for prevention of dislocation or fall-out of the stent.

In another embodiment of the stent-retention mechanism, a retention mechanism is preferably formed in the distal-end side of the crimped stent, when an external force is applied to the stent for movement toward the distal-end side.

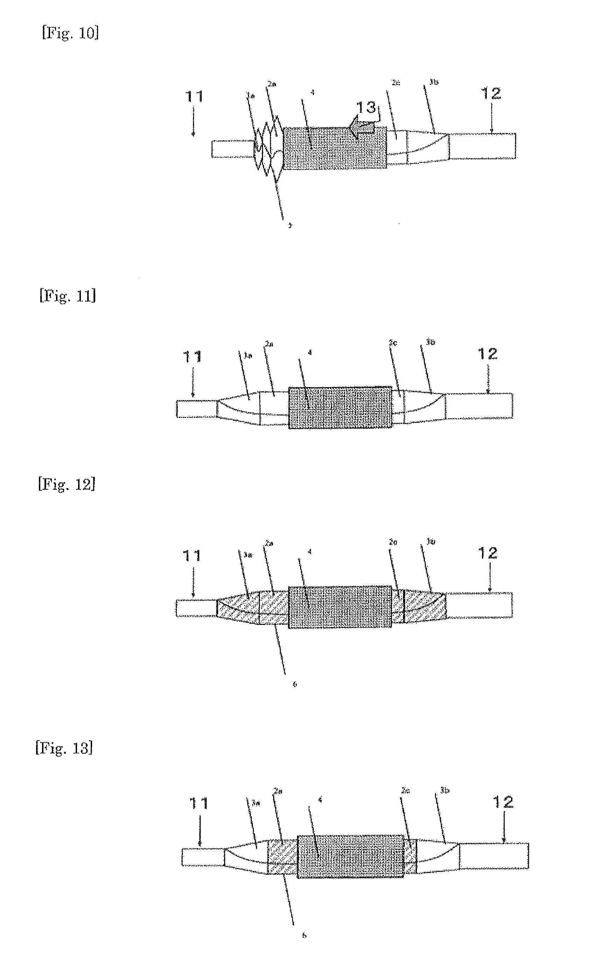

As shown in Figure 9, the stent-retention mechanism is preferably formed in at least part of the region within the straight-tube region where the crimped stent is not placed and the tapered region. As shown in Figure 10, the stent-retention mechanism is preferably a stopper that prevents fall-off of the stent with at least one pleat formed thereon.

### (3) Stent crimping

To crimp the stent, the balloon is folded into multiple parts and wound around the inner tube of the stent in the catheter axial direction. The balloon can be folded into two or more parts by a method known by those who are skilled in the art. As for the winding method, if the balloon is folded into two, the folded balloon may be wound in the same rotating direction (S wrapping) or in the opposite rotating direction (C wrapping). If the balloon is folded into three or more parts, it is generally wound in the same direction.

As shown in Figure 5, if the balloon is folded into at least two and wound around the catheter in the axial direction, the stent is preferably crimped on the straight-tube region, leaving the distal-end sided region 2a and proximal-end sided region 2c of the straight-tube region not in contact with the stent outside the crimped stent. More preferably as shown in Figure 11, of the distal-end sided region 2a and the proximal-end sided region 2c of the straight-tube region not in contact with the stent, the distal-end sided region 2a is longer. The tapered region is preferably longer than the straight-tube region where stent is not placed on at least one side of the wound stent, and more preferably, the distal-end sided region has such state.

It is preferable in crimping the stent around the delivery catheter, to reduce the diameter uniformly over the entire circumferences by applying a uniform force from outside the stent. Excessively large compression force leads to difficulty in movement of the compressed stent and reduction in diameter of the stent after crimping. It is more preferable that the stent is crimped with an external force of 50 N or more.

### (4) Other variations of stent-retention mechanism

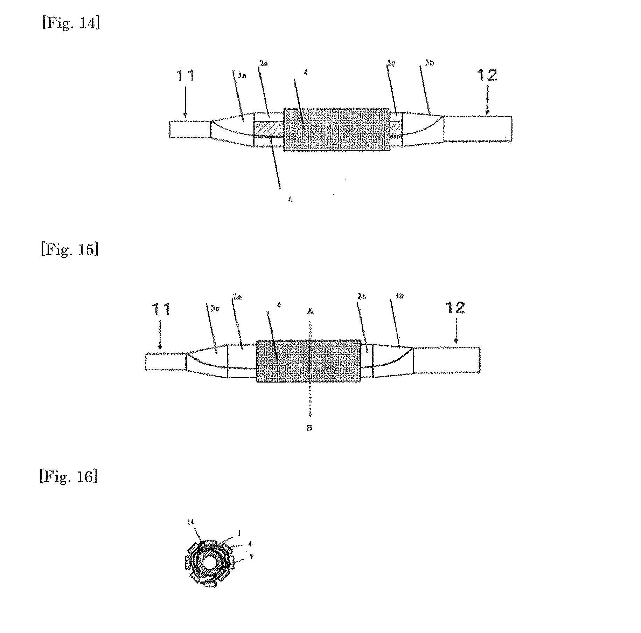

Some modified embodiments of the stent-retention mechanism will be described below with reference to drawings. As shown in Figures 12, 13 and 14, the stent-retention strength may be reinforced by frictional force due to addition of a friction layer 6 formed between the balloon and the stent. In such a case, at least one layer having a friction coefficient larger than that of a balloon is preferably provided. As shown in Figures 13 and 14, a friction layer 6 may be formed at least on part of the balloon.

As shown in Figure 15, the stout-retention strength may be reinforced by catch of the balloon between the stent struts, in addition to the frictional force above. In such a case, as shown in Figure 16, the vertexes 14 of the blades of the folded balloon are placed between the struts of the crimped stent. In any case, the stent-retention mechanism is formed only when a force moving the stent in the catheter axial direction is applied.

### (5) Examples of the materials and shapes of balloon

The material for the medical balloon 1 does not have adverse effects on the advantageous effects of the present invention at all, if it is a material allowing biaxial stretching, and examples thereof for use include polyolefins, polyolefin elastomers, polyesters, polyester elastomers, polyamides, polyamide elastomers, polyurethanes, polyurethane elastomers and the like, and to make the stent thin and flexible and yet strong enough to withstand the pressure for expansion of the stent, use of a polyester, a polyester elastomer, a polyamide, or a polyamide elastomer is preferable. The balloon preferably has a shape having a taper angle of 30° or more, and more preferably a taper angle of 50°. The film thickness is preferably thicker in the straight-tube region than in the tapered regions.

### (6) Preparation of balloon

The balloon is prepared with a tube called parison by blow molding of applying a pressure in a heated mold. The method of preparing the balloon is not limited to this process, and the balloon may be prepared by a method of applying a resin around a mold by dipping. The balloon-producing methods include dip molding, blow molding and others, and a preferable method may be selected, but blow molding is preferable to make a balloon have a pressure-withstanding strength sufficient for expansion of the stent.

For example, a tubular parison having a desired dimension is first prepared, for example, by extrusion molding. The tubular parison is placed in a mold having the shape identical with that of the balloon and is stretched in the axial direction and also in the diameter direction by biaxially stretching process to give a balloon having the shape identical with that of the mold. Stretching in the axial direction may be carried out simultaneously with or before/after stretching in the diameter direction, and annealing treatment may be carried out for stabilization of the shape and size of the balloon.

### (7) Examples of the materials for inner tube, outer tube, and hub

The material for the inner tube 7 does not have any influence on the advantageous effects of the present invention and thus is not thus particularly limited. If the device has a coaxial structure having a coaxial double tube, polyolefins, polyolefin elastomers, polyesters, polyester elastomers, polyamides, polyamide elastomers, polyurethanes, polyurethane elastomers, and others can be used for the inner tube 7, but, because the guide wire lumen is formed by the internal surface of the inner tube 7, considering the slidability of the guide wire passing therein, use of polyethylene, in particular use of high-density polyethylene, is preferable.

The inner tube 7 may have a multilayered structure having an innermost layer of high-density polyethylene for improvement in slidability of the guide wire, and an outermost layer of a material that can adhere to or fuse with the balloon 1. For further improvement in slidability of the guide wire, the internal surface of the inner tube 7 may be finished with a lubricant such as silicone or polytetrafluoroethylene. If the device has, for example, a biaxial structure having two parallel tubes or other structures, preferable materials for these tubes can be selected according to the description above.

The material for the outer tube 8 is not particularly limited either, similarly to the material for the inner tube 7, and polyolefins, polyolefin elastomers, polyesters, polyester elastomers, polyamides, polyamide elastomers, polyurethanes, polyurethane elastomers, and the like are usable.

The materials preferably used for the hub 9 include polycarbonates, polyamides, polyurethanes, polysulfones, polyarylates, styrene-butadiene copolymers, polyolefins, and the like.

The method of bonding the balloon to the outer and inner tubes is not particularly limited, and any known bonding method, such as adhesion with an adhesive agent or fusion, may be used. The composition, the chemical structure and the hardening method of the adhesive agent used are not limited. From the points of composition and chemical structure, urethane-based, silicone-based, epoxy-based, cyanoacrylate-based and other adhesive agents are usable, and from the point of hardening method, two-liquid mixture-type adhesives, UV-hardening adhesives, water-hardening adhesives, heat-hardening adhesives, radiation-hardening adhesives and others are usable.

However, if an adhesive agent is used, it is preferable to use an adhesive agent having a post-hardening hardness at a level preventing discontinuous change in rigidity of the junction areas between the proximal end-sided sleeve region of balloon and the outer tube, and between the distal end-sided sleeve region of balloon and the inner tube, and such an adhesive agent can be selected according to the rigidity of the balloon, and the outer and inner tubes.

### (8) Stent

The stent according to the present invention (e.g., body cavity-expanding stent) is not limited if it is a balloon-expandable stent, and the material thereof is not particularly limited either, and stainless steels such as SUS316L, cobalt chromium alloys, and the like can be used. The design of the stent is not particularly limited either.

### (9) Examples of the materials for hydrophilic coating

The external surface of catheter may be subjected to hydrophilic coating for facilitating insertion thereof into blood vessels or into guide catheter. It is preferable to apply hydrophilic coating on at least a part of the shaft of catheter in contact with blood to make it more lubricating when in contact with blood. The kind of the hydrophilic coating does not restrict the advantageous effects of the present invention; hydrophilic polymers such as polyethylene glycol, polyacrylamide, and polyvinylpyrrolidone are used preferably; and the coating method is not limited either.

In the case of the delivery catheter above, because presence of a hydrophilic coating on the balloon surface facilitates fall-off of the stent, it is also possible to not apply the hydrophilic coating on the balloon surface, to remove the hydrophilic coating only from balloon, or to form a layer having a higher friction coefficient such as of urethane or rubber over the hydrophilic coating for further increase in frictional resistance.

### (10) Stent delivery catheter

An example of the "stent delivery catheter" according to the present invention is a stent delivery catheter having the catheter described below in Example 1 connected thereto. In addition to the catheter shown in Example 1, any catheter produced by a common method known to those who are skilled in the art may be used. The catheter preferably has a soft shaft. The shaft may have a fluid-supplying port on the proximal-end side thereof.

### Examples

Hereinafter, the present invention will be described more in detail with reference to Examples, but it should be understood that the present invention is not particularly restricted by these Examples and others.

### (Example 1)

A catheter was prepared according to the procedure described below. The entire structures of the catheter of Example 1 and its explanation are the same as those exemplified in Figures 1 and. 3 and described above in the embodiments.

A tubular parison (internal diameter: 0.45 mm, external diameter: 0.87 mm) was prepared with a polyamide elastomer (trade name: PEBAX7233SA01; manufactured by Elf Atochem) by extrusion molding; and a balloon having a straight-tube region, with an external diameter of 3.00 mm, a length of 20.5 mm, and a taper angle of 50°, having thickness 16µm at around the center, a distal end-sided tapered region having a thickness of 19µm at around the center, and a proximal end-sided tapered region having a thickness of 19µm at around the center was then prepared with the parison by biaxial-stretching blow molding. The thicknesses of the distal end-sided tapered region, the straight-tube region, and the proximal end-sided tapered region respectively at around the centers were determined by using a micrometer.

The inner tube (internal diameter: 0.42 mm, external diameter: 0.56 mm) and the outer tube (internal diameter: 0.76 mm, external diameter: 0.90 mm) were prepared with a polyamide elastomer (trade name: PEBAX7233SA01; manufactured by Elf Atochem) by extrusion molding.

The stent was prepared by machining a SUS316L tube (internal diameter: 1.80 mm, external diameter: 2.05 mm) into a desired pattern by laser processing and electrolytically polishing the resulting tube.

The outer tube and the balloon were bonded to each other by heat fusion, and the inner tube was inserted into the tube to give a coaxial double tube with the outer tube. The balloon and the inner tube were then bonded to each other by heat fusion in the balloon distal end-sided sleeve region, while the distal end of the inner tube is extending inside the balloon. A core material in an arbitrary dimension that was previously coated with a high-lubricity material such as molding polytetrafluoroethylene was used as needed for bonding, in formation of the inflation lumen or the guide wire lumen. A hub was connected to the proximal-end side of the outer tube to give a delivery catheter. The delivery catheter was subjected to hydrophilic coating; the hydrophilic coating on the balloon surface was removed; and a urethane layer was formed thereon.

The delivery catheter was folded into the triset shape (shape of balloon folded into three blades) under reduced pressure and a stent with a diameter reduced to 60N was placed thereon. The configuration of the stent-retention mechanism obtained in Example 1 is the same as the configuration exemplified in Figure 12. The length of the distal-end sided region 2a of the straight-tube region where the stent is not placed was 1.1 mm, while the length of the proximal end-sided region 2c of the straight-tube region was 0.25 mm. The length of the distal end-sided taper 3a was 2 mm, which was longer than the distal-end sided region 2a of the straight-tube region where the stent was not placed.

### (Comparative Example 1)

A delivery catheter was prepared in a manner similar to Example 1; the balloon was folded into the triset state, and a crimped stent was placed thereon before use. In Comparative Example 1, the stent was placed on the middle to the distal end area of the straight tube and crimped, so that the proximal-end sided region of the straight-tube region where the stent was not placed became longer than the distal-end sided region of the straight-tube region where the stent was not placed.

### (Comparative Example 2)

A delivery catheter was prepared in a manner similar to Example 1; processings to the removal of the hydrophilic coating on the balloon were carried out in Comparative Example 2; the balloon was folded into the triset state; and a crimped stent was placed thereon before use. The stent was further placed on the middle to distal-end side of the straight tube and crimped, so that the proximal-end sided region of straight-tube region where the stent is not placed became longer than the distal-end sided region of straight-tube region where the stent is not places.

### (Evaluation of trackability performance by using simulated blood vessel plate)

The efficiency in insertion operation of each sample thus prepared (Example 1. or Comparative Example 1) into a simulated blood vessel was evaluated. In evaluation, used was a system consisting of a guide catheter (Launcher: 6Fr, JL3.5, manufactured by Medtronic), a hemostasis valve, and a guide wire (Neo's Intermediate: manufactured by Asahi Intecc) immersed in water at 37°C and additionally a simulated blood vessel plate, into which the guide catheter was inserted, wherein water is circulated into the internal space of the guide catheter, the hemostasis valve, and the plate. A sample was inserted through the opening of the hemostasis valve and the proximal end of the sample was chucked to a load meter, and the resistance during passage through the plate was determined.

### (Evaluation of stent fall-off resistance)

Each sample prepared was pulled toward the proximal end by a tensile test machine, while the sample was held at the stent proximal end region, and the strength thereof at least during movement or fall-off of the stent was determined. For example, if the stent moved and then fell off, the strength during movement of the stent was determined.

### (Evaluation results)

As shown in Table 1, comparison of the passage loads of the stents into the simulated blood vessel plate in inventive Example 1 and Comparative Example 1 shows that there is almost no difference among the Example in which the stent-retention mechanism was formed, the Comparative Example 1 in which the stent was simply crimped, and the Comparative Example 2 in which the urethane layer was not formed. On the other hand, in the stent-fall-off strength, application of a force of approximately 1.5 N resulted in formation of a stent-retention mechanism, which showed high retention strength as stopper, in Example 1, but the stent-retention mechanism was not formed, resulting in fall-off of the stent at low force in Comparative Example 1. The retention strength was further lower at 0.5 N in Comparative Example 2. The difference therein between Example 1 and Comparative Example 1 was about 1 N, and the strength was higher when the stent-retention mechanism was formed. These results demonstrated that application of a force on the stent for movement toward the distal-end side lead to formation of a stent-retention mechanism, which resulted in prevention of fall-off of the stent and preservation of the trackability performance because no special mechanism was formed additionally.

**[Table 1]**

| | Trackability maximum load (gf) | Stent-retention strength (N) |
|---|---|---|
| Example 1 | 46 | 2.5 |
| Comparative Example 1 | 42 | 1.5 |
| Comparative Example 2 | 43 | 0.5 |

## Claims

1. stent delivery system comprising a delivery catheter having a balloon and a stent, **characterized in that** the balloon has a straight-tube region and a tapered region formed in at least one of the distal and proximal end-sided areas thereof, and at least a part of the balloon forms a steut-retention mechanism.

2. The stent delivery system according to Claim 1, wherein the stent-retention mechanism is not formed when the stent is placed in the delivery catheter as the diameter thereof is reduced, and is formed when a force for moving the stent in the catheter axial direction is applied.

3. The stent delivery system according to Claim 2, wherein the stent-retention mechanism returns back to its original state when the force for moving the stent in the catheter axial direction is removed.

4. The stent delivery system according to Claim 2 or 3, wherein the stent-retention mechanism is formed on at least one terminal area of the distal- and proximal-end sides of the stent placed as crimped.

5. The stent delivery system according to Claim 4, wherein the stent-retention mechanism is formed on at least an area of the straight-tube region of the balloon where the crimped stent is not placed and the tapered region.

6. The stent delivery system according to Claim 4, wherein the stent-retention mechanism serves as a stopper preventing fall-off of the stent by forming at least one pleat.

7. The stent delivery system according to Claim 4, wherein the tapered region is longer than the region of the balloon straight-tube region where the stent is not placed, on at least one terminal area of the stent distal- and proximal-end sides

8. The stent delivery system according to Claim 4, wherein the stent-retention mechanism is formed on the distal-end side of the crimped stent, when a force for moving the stent in the distal-end side is applied.

9. The stent delivery system according to Claim 4, wherein the straight-tube region of the balloon has regions where the stent is not placed both on the distal- and proximal-end sides, and the distal end-sided region is longer than the proximal end-sided region.
